# EUROPÄISCHE PATENTSCHRIFT

(11) **EP 0 522 577 B1**
(45) Veröffentlichungstag und Bekanntmachung des Hinweises auf die Patenterteilung: **11.10.1995**
(21) Anmeldenummer: 92111765.1
(22) Anmeldetag: 10.07.1992
(51) Int. Cl.: C07C 209/84

(54) **Verfahren zur Herstellung von reinem 3,3',4,4'-Tetraaminobiphenyl**
Process for the preparation of pure 3,3',4,4'-tetraaminobiphenyl
Procédé pour la préparation de 3,3',4,4'-tetraaminbiphenyle

(30) Priorität: 12.07.1991 DE 4123033
(43) Veröffentlichungstag der Anmeldung: 13.01.1993
(73) Patentinhaber: HOECHST AKTIENGESELLSCHAFT, 65926 Frankfurt am Main (DE)
(72) Erfinder: Vorwerk, Edgar, Dr., W-6233 Kelkheim (Taunus) (DE)

(56) Entgegenhaltungen:
- EP-A- 0 061 168
- US-A- 3 390 180

## Beschreibung

Die Erfindung betrifft ein Verfahren zur Herstellung von reinem 3,3',4,4'-Tetraaminobiphenyl (Abkürzung TAB) in hoher Ausbeute, welches durch sparsamsten bzw. ohne Zusatz von Hilfsstoffen und mit oder ohne Rückfluß von Umlösewasser durchgeführt wird, wodurch deutlich verbesserte Ausbeuten an reinem, qualitativ hochwertigem TAB erhalten werden.

3,3',4,4'-Tetraaminobiphenyl ist ein wichtiges Ausgangsmaterial für die Herstellung von beispielsweise hochtemperaturbeständigen Polymeren, wie den Polybenzimidazolen, wie beispielsweise in den US-PS 2,895,948, 3,174,947, 3,578,644, 4,431,796, 4,452,967 beschrieben. Die Herstellung solcher Polymeren stellt hohe Ansprüche an das verwendete TAB hinsichtlich Reinheit, Farbe und Restgehaltsmengen. Die Herstellung von 3,3',4,4'-Tetraaminobiphenyl erfolgt durch Umsetzung von 3,3'-Dichlorbenzidin mit Ammoniakwasser im Druckreaktor bei 180-220°C in Gegenwart von Kupferkatalysatoren. Diese Austauschreaktion von Chlorgegen Aminogruppen ist mehrfach beschrieben worden, wobei unterschiedliche Kupferkatalysatoren zum Einsatz kommen.

Gemäß dem in der US-PS 3,865,876 beschriebenen Verfahren wird als Katalysator Kupfer(l)-chlorid verwendet. In der US-PS 3,943,175 wird die Herstellung des TAB unter Verwendung eines Katalysatorgemisches aus Kupferpulver und Kupfer(l)-chlorid beschrieben. Katalysatorgemische aus Kupferpulver und Kupfer(l)-chlorid (CuCl) sind für die Umsetzung besonders geeignet und werden auch gemäß den neueren Verfahrens- und Herstellungsbeschreibungen verwendet.

Sowohl in der DE-PS 3 111 470 C2 als auch in der JA-OS Sho-60-158146 werden Herstellungsverfahren für TAB mit derartigen Katalysatorgemischen beschrieben: 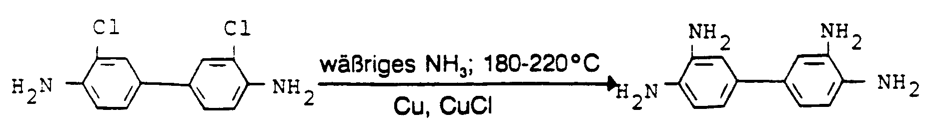
Das aus der Umsetzung, d.h. nach Abtrennung des kupfersalzhaltigen Ammoniakwassers, erhaltene rohe 3,3',4,4'-Tetraaminobiphenyl ist für die vorgesehene Verwendung ungeeignet und muß einem Reinigungsprozeß unterworfen werden, um ein TAB zu erhalten, das für die Verarbeitung zu Polymeren (z.B. Polybenzimidazolen) hinsichtlich der Farbqualität, dem Restkupfergehalt und störender Nebenprodukte geeignet ist. Daher galt der Reinigung des rohen TAB immer besondere Aufmerksamkeit und in allen vorstehend zitierten Patentschriften werden detailliert Reinigungsverfahren mit dem Ziel beschrieben, weiterverarbeitbares Material herzustellen.

Nachstehend wird der Stand der Technik hinsichtlich Herstellung von reinem TAB erläutert; ferner werden die Ergebnisse sowie die spezifischen Nachteile dargelegt. Wie vorstehend bereits erwähnt, hat sich bei der Herstellung von 3,3',4,4'-Tetraaminobiphenyl aus 3,3'-Dichlorbenzidin ein Katalysatorgemisch aus Kupferpulver und CuCl bewährt, so daß alle neueren Herstellverfahren (siehe die zitierten Patentschriften) damit arbeiten. Eine wesentliche Zielsetzung besteht darin, das zugefügte ionogene und metallische Kupfer weitgehend aus dem TAB zu entfernen, um möglichst geringe Restkupfergehalte zu erzielen, die auch für die erwünschte gute Farbqualität von Bedeutung sind.

In der US-PS 3,943,175 wird das aus der Umsetzung erhaltene, rohe TAB zunächst von der ammoniakalischen Mutterlauge abgetrennt und mit Wasser nachgewaschen. Mit verdünnter Schwefelsäure wird das säureunlösliche TAB-Dihydrosulfat hergestellt, aus dem durch Umsetzung mit einer Base das freie TAB wiedergewonnen wird. Anschließend wird das TAB aus Wasser unter Zusatz von Aktivkohle und Diatomeenerde umkristallisiert. Hierzu wird in ca. 67-facher Wassermenge unter Zusatz von 40 % Aktivkohle und 15 % Diatomeenerde (jeweils bezogen auf die umzulösende TAB-Menge) gearbeitet.

Nach diesem Herstellungs- und Reinigungsverfahren werden 51,9 % 3,3',4,4'-Tetraaminobiphenyl mit einem Schmelzpunkt von 175-181°C erhalten. Der Restkupfergehalt des Materials beträgt 0,07 % Kupfer.

In der DE-PS 3 111 470 C2 wird eine verbesserte Aufarbeitung von rohem TAB beschrieben, das durch Umsetzung von 3,3'-Dichlorbenzidin mit wäßrigem Ammoniak in Gegenwart eines Katalysatorgemisches aus Kupferpulver und CuCl erhalten wurde. Auch dieses Reinigungsverfahren gliedert sich in zwei Verfahrensschritte. Zunächst wird das rohe TAB von der kupferhaltigen, ammoniakalischen Reaktionsmutterlauge abgetrennt und dann mit ca. 25 %igem Ammoniakwasser und anschließend mit Wasser gewaschen, um lösliche Kupferverbindungen aus dem rohen TAB zu entfernen. Die Wasserwäsche dient der Entfernung von Ammoniakresten. Für die Ammoniakwäsche werden, bezogen auf das rohe TAB, ca. 2-4 Teile etwa 25 %iges Ammoniakwasser eingesetzt.

Im zweiten Verfahrensschritt wird helles, farblich lagerstabiles und nur noch Kupferspuren enthaltendes TAB durch Umkristallisation aus Wasser hergestellt. Diese Umkristallisation wird unter einer Schutzgasatmosphäre unter Zusatz von Aktivkohle oder Diatomeenerde sowie einem wasserlöslichen Reduktionsmittel (Natriumdithionit) durchgeführt. Bezogen auf das rohe TAB wird ca. die 29-fache Wassermenge unter Zusatz von ca. 20 % Aktivkohle und ca. 2 % Natriumdithionit eingesetzt.
Derart werden 71,3 % d. Th. 3,3',4,4'-Tetraaminobiphenyl mit einem Schmelzpunkt von 177-180°C erhalten. Der Restkupfergehalt des Materials beträgt ca. 0,005 %, die Farbe ist schwach sandfarben.

Das Verfahren der JA-OS Sho-60-158146 stellt gegenüber dem der DE-PS 3 111 470 C2 kaum einen Fortschritt dar. Beschrieben wird in der JA-OS ein Reinigungsverfahren für rohes TAB, das durch Umsetzung von 3,3'-Dichlorbenzidin mit wäßrigem Ammoniak in Gegenwart eines Katalysatorgemisches aus Kupferpulver und CuCl erhalten wurde. Sehr ähnlich dem Verfahren der DE-PS 3 111 470 C2 ist das in der genannten JA-OS beschriebene Verfahren in zwei Schritte untergliedert: Nach Abkühlung des Reaktionsgemisches wird das rohe TAB von der kupferhaltigen, ammoniakalischen Reaktionsmutterlauge abgetrennt und mit ca. 30 %igem Ammoniakwasser und anschließend mit Wasser gewaschen. Die Ammoniakwäsche dient zur Abtrennung löslicher Kupferverbindungen, während durch die anschließende Wasserwäsche Ammoniakreste entfernt werden. Für die Ammoniakwäsche werden, bezogen auf das rohe TAB, ca. 4 Teile etwa 30 %iges Ammoniakwasser eingesetzt.

Im zweiten Verfahrensschritt der JA-OS wird das rohe TAB durch eine Umkristallisation aus Wasser gereinigt und helles, farblich stabiles und nur noch Kupferspuren enthaltendes TAB erhalten. Die Umkristallisation wird unter einer Schutzgasatmosphäre sowie unter Zusatz von Aktivkohle, Eisenchlorid und Hydrazin durchgeführt.

Bezogen auf das rohe TAB wird die ca. 73-fache Wassermenge eingesetzt; ferner werden ca. 30 % Aktivkohle, ca. 0,5 % Eisenchlorid und ca. 7 % Hydrazin zugegeben.
Derart werden 75,6 % d. Th. 3,3',4,4'-Tetraaminobiphenyl (bezogen auf Dichlorbenzidin) mit einem Schmelzpunkt von 177-178°C erhalten. Der Restkupfergehalt des TAB beträgt ca. 0,001 % (10 ppm), die Farbe ist weißlich erdfarben.

Die Reinigungsverfahren, wie sie vorstehend beschrieben wurden, stellen auf diesem Gebiet zwar einen deutlichen Fortschritt dar, sie befriedigen aber bei einer technischen Durchführung nicht.
Die Umsetzung von 3,3'-Dichlorbenzidin mit Ammoniakwasser im Druckreaktor bei 180-220°C in Gegenwart von Kupferpulver und Kupfer(l)-chlorid als Katalysatoren verläuft praktisch vollständig, so daß in der Reaktionsmutterlauge bzw. im rohen 3,3',4,4'-Tetraaminobiphenyl nur noch Spuren an 3,3'-Dichlorbenzidin vorhanden sind (deutlich unter 100 ppm; häufig unterhalb 10 ppm).

Bei einer derart gut verlaufenden Umsetzung können Ausbeuten an isoliertem Endprodukt von ca. 71-75 % nicht befriedigen, zumal die wesentliche Reinigungsstufe eine Umkristallisation ist, bei der diese Ausbeuteverluste nicht auftreten sollten. Allerdings ist die Aufarbeitung nach Maßgabe hoher Qualitätsanforderungen and das Endprodukt durchzuführen.

Die beschriebenen Ergebnisse wurden mit bereits optimierten Umkristallisationsverfahren erzielt, bei denen die verwendeten Hilfsstoffmengen erfindungsgemäß exakt angegeben werden.

Nach der DE-PS 3 111 470 C2 sind zu verwenden:

| | |
|---|---|
| - an Adsorbens (z.B. Aktivkohle) vorzugsweise 15-25 Gew.-% | 10-30 Gew.-%, |
| - an Reduktionsmittel (z.B. Natriumdithionit) vorzugsweise 2-3 Gew.-%. | 2-10 Gew.-%, |

Alle Gewichtsprozentangaben beziehen sich dabei auf trockenes, rohes TAB, dessen Ausbeute, bezogen auf eingesetztes 3,3'-Dichlorbenzidin, mit 93,4 % d. Th. angegeben wird.

Nach der JA-OS Sho-60-158146 sind zu verwenden:

| | |
|---|---|
| - an Aktivkohle | 5-50 Gew.-%, vorzugsweise 10-30 Gew.-% |
| - an Eisenchlorid | 0-10 Gew.-%, vorzugsweise 1-5 Gew.-% |
| - an Hydrazin | 3-30 Gew.-%, vorzugsweise 10-20 Gew.-%. |

Alle Gewichtsprozentangaben beziehen sich auch hier auf trockenes, rohes TAB, dessen Ausbeute, bezogen auf eingesetztes 3,3'-Dichlorbenzidin, mit 94,1 % d. Th. angegeben wird.

Neben der unbefriedigenden Endproduktausbeute stören bei den bisherigen Verfahren auch die hohen Abfallmengen bei Verwendung großer Aktivkohlemengen.
Die Verwendung von Hydrazin als Reduktionsmittel bei der Umlösung ist, bei dessen cancerogenem Potential, ohnehin unter heutiger Sicht als unsinnig zu bezeichnen.

Es bestand somit der Bedarf nach einem Reinigungsverfahren für qualitativ hochwertiges TAB, das bessere Ausbeuten bei möglichst verringerten Abfallmengen liefert.

Es wurde nun überraschenderweise gefunden, daß man reines, qualtitativ hochwertiges 3,3',4,4'-Tetraaminobiphenyl (TAB) in hoher Ausbeute aus rohem TAB, welches in bekannter Weise durch Ammonolyse von 3,3'-Dichlorbenzidin in Gegenwart von Kupferpulver und CuCl hergestellt und anschließend mit Ammoniakwasser und Wasser gewaschen wurde, herstellen kann, indem man das derart vorbehandelte TAB in Wasser in Gegenwart von 0 bis 5 Gew.-% Aktivkohle und von 1 bis 2 Gew.-% eines wasserlöslichen Reduktionsmittels, jeweils bezogen auf das rohe TAB, bei Temperaturen von 100°C bis 140°C, vorzugsweise von 105° bis 125°C, unter einer Schutzgasatmosphäre umlöst (umkristallisiert).

Für das erfindungsgemäße Verfahren wird rohes TAB, wie in der Literatur beschrieben, hergestellt, wozu ein Katalysatorgemisch aus Kupferpulver und Kupfer(l)-chlorid verwendet wird.
Nach Isolierung des rohen TAB durch Abtrennung der Reaktionsmutterlauge wird das Rohmaterial (TAB) literaturgemäß mit Ammoniakwasser (Konzentration 10-35 % NH₃) gewaschen, um die anhaftenden, löslichen Kupfersalze zu entfernen, und anschließend mit Wasser nachgewaschen, um anhaftende Ammoniakreste zu entfernen.
Derart wird ein TAB mit einem Kupfergehalt von 0,2-2,0 % erhalten, wie in der DE-PS 3 111 470 C2 bzw. JA-OS Sho-60-158146 beschrieben.

Die erfindungsgemäße Umlösung (Umkristallisation) erfolgt ohne Zwischentrocknung des vorbehandelten TAB durch Eintragung der feuchten Ware unter Stickstoff in den Umlösekessel.

Bezogen auf das rohe, trockene TAB (93-94 % d. Th., bezogen auf 3,3'-Dichlorbenzidin) werden 0-5 Gew.-%, bevorzugt 3 bis 4,5 Gew.-%, Aktivkohle der Umlösung (Umkristallisation) zugesetzt. Auf den Zusatz von Aktivkohle kann auch ganz verzichtet werden. Die Aktivkohle dient im vorliegenden Fall weniger als Adsorbens für abzutrennende, störende Nebenprodukte, sondern mehr als Filterhilfsstoff für die wasserunlöslichen, nichtumgesetzten Kupferpulverreste aus dem Katalysator sowie unlösliches, organisches Material. Aus diesen Gesichtspunkten kann auf den Aktivkohlezusatz verzichtet werden; aus verfahrenstechnischen Gründen, nämlich wegen einer guten Filtration, ist ein Zusatz von 3 bis 4,5 Gew.-% Aktivkohle bei der Umlösung aber zweckmäßig. Damit ergibt sich, daß auch andere Filtrationshilfsstoffe (wie beispielsweise Silicagel) in entsprechender Menge und Anwendung einsetzbar sind.

Die Umlösung wird unter einer Schutzgasatmosphäre, beispielsweise einer Stickstoffatmosphäre durchgeführt.

Zur Vermeidung jeglicher oxidativer Bedingungen wird der Umlösung ein wasserlösliches Reduktionsmittel zugesetzt. Bewährt hat sich die Verwendung von Natriumdithionit (Na₂S₂O₄); es können aber auch andere Alkalimetalldithionite oder Alkalimetallsulfite, wie Natriumdithionit oder Natriumsulfit, oder Hydrazin verwendet werden.

Bezogen auf das rohe TAB sind nur 1 bis 2 Gew.-% Alkalimetalldithionit oder -sulfit einzusetzen.

Die benötigte Wassermenge ergibt sich aus der Löslichkeit des TAB bei der Umkristallisationstemperatur. Das Umlösewasser kann im Kreis gefahren und bei der nächsten Umlosung erneut verwendet werden. Vorteilhaft ist eine Teilrückführung des Umlösewassers bei gleichzeitiger Teilausschleusung als Abwasser.

Die Umkristallisation wird im Verlauf von 0,5-1,5 Stunden bei Temperaturen von 110 bis 120°C durchgeführt.

Beim erfindungsgemäßen Verfahren werden Ausbeuten an reinem TAB von 88,2 % d. Th., bezogen auf 3,3'-Dichlorbenzidin, erhalten. Dies stellt gegenüber dem Stand der Technik eine Steigerung von über 12 % dar, was für ein bereits ausgearbeitetes Verfahren einen wesentlichen Fortschritt bedeutet.

Die erhaltenen Produktqualitäten sind den literaturbekannten Produktqualitäten ebenbürtig. Das reine TAB, hergestellt nach dem erfindungsgemäßen Verfahren, ist weißlich bis hell sandfarben und hat einen Restgehalt an Kupfer von nur noch 0,001 % (10 ppm); teilweise sogar noch weniger. Der Festpunkt beträgt 176-178°C.

Diese Ergebnisse werden bei deutlich geringeren Hilfsstoffzusätzen als bisher üblich erzielt, so daß zwangsläufig bei dem erfindungsgemäßen Verfahren auch die Abfallmengen deutlich vermindert sind.

### Beispiel

In einem Druckreaktor aus V2A-Stahl werden 60 kg 3,3'-Dichlorbenzidin, 7,5 kg CuCl, 2 kg Kupferpulver und 450 kg ca. 30 %iges Ammoniakwasser, das 25 kg Ammoniumchlorid enthält, unter einer Stickstoffatmosphäre vorgelegt. Das Reaktionsgemisch wird 8 Stunden auf 200-210°C erhitzt, wobei sich zunächst ein Druck von ca. 65 bar einstellt, der im Reaktionsverlauf auf 55-60 bar abfällt. Nach erfolgter Reaktion wird der Reaktorinhalt abgekühlt, sodaß das rohe 3,3',4,4'-Tetraaminobiphenyl aus der Reaktionsmutterlauge ausfällt und bei Raumtemperatur über einen Filter oder eine Zentrifuge unter Stickstoff abgetrennt werden kann. Dieses Rohprodukt enthält noch lösliche Kupfersalze, die weitgehend durch zweifaches Waschen mit insgesamt 220 kg 25 %igem Ammoniakwasser entfernt werden. Eine sich anschließende Wasserwäsche mit 140 l Wasser entfernt dann Ammoniakreste. Auch diese Verfahrensschritte werden unter Stickstoff durchgeführt.
Derart wird ein rohes TAB erhalten, dessen Restkupfergehalt zwischen 0,2 und 2,0 % liegt. Nicht umgesetztes 3,3'-Dichlorbenzidin ist nur noch in Spuren vorhanden (weniger als 100 ppm; meist weniger als 10 ppm). Dieses rohe TAB wird nicht isoliert, sondern direkt unter Stickstoff der sich anschließenden Umlösung zugeführt.

Das aus der Umsetzung und der anschließenden Ammoniak- und Wasserwäsche erhaltene rohe TAB wird in 1200 l Wasser eingetragen. In dieser Menge sind 600 l rückgeführtes Umlösewasser aus der vorangegangenen Umkristallisation enthalten. Hinzugefügt werden 2 kg Aktivkohle sowie als wasserlösliches Reduktionsmittel 0,6 kg Natriumdithionit (Na₂S₂O₄). Die Umlösung erfolgt bei 110-120°C während 45 min. unter einer Stickstoffatmosphäre. Anschließend wird die noch heiße Lösung über einen Filter gegeben, um die Aktivkohle zusammen mit unlöslichem organischen Material sowie den Kupferpulverresten des Katalysators abzutrennen. Der Filterkuchen wird zweifach mit je 10 l heißem Wasser nachgewaschen, das zu dem Filtrat gegeben wird. Die vereinigten Filtrate werden auf Raumtemperatur (ca. 25°C) abgekühlt und das ausfallende, gereinigte TAB abfiltriert oder abzentrifugiert. Nach sorgfältiger Trocknung bei 50-70°C unter einem Wasserstrahlvakuum werden 44,8 kg TAB erhalten, was einer Ausbeute von 88,2 % der Theorie, bezogen auf 3,3'-Dichlorbenzidin, entspricht.
Das erhaltene Produkt weist einen Festpunkt von 176-178°C auf und ist weißlich bis hell sandfarben.
Der Restkupfergehalt liegt bei 0,001 % (10 ppm) teilweise noch darunter.

## Patentansprüche

1. Verfahren zur Herstellung von reinem, qualtitativ hochwertigen 3,3',4,4'-Tetraaminobiphenyl (TAB) in hoher Ausbeute aus rohem TAB, welches in bekannter Weise durch Ammonolyse von 3,3'-Dichlorbenzidin in Gegenwart von Kupferpulver und CuCl hergestellt und anschließend mit Ammoniakwasser und Wasser gewaschen wurde, dadurch gekennzeichnet, daß man das derart vorbehandelte TAB in Wasser in Gegenwart von 0 bis 5 Gew.-% Aktivkohle und von 1 bis 2 Gew.-% eines wasserlöslichen Reduktionsmittels, jeweils bezogen auf das rohe TAB, bei Temperaturen von 100° bis 140°C unter einer Schutzgasatmosphäre umlöst.

2. Verfahren nach Anspruch 1, dadurch gekennzeichnet, daß man das Umlösen bei Temperaturen von 105 bis 125°C durchführt.

3. Verfahren nach mindestens einem der Ansprüche 1 und 2, dadurch gekennzeichnet, daß man als wasserlösliches Reduktionsmittel ein Alkalimetalldithionit oder -sulfit verwendet.

4. Verfahren nach mindestens einem der Ansprüche 1 bis 3, dadurch gekennzeichnet, daß man als Reduktionsmittel Natriumdithionit verwendet.

5. Verfahren nach mindestens einem der Ansprüche 1 bis 4, dadurch gekennzeichnet, daß man die Umlosung unter Stickstoff vornimmt.

6. Verfahren nach mindestens einem der Ansprüche 1 bis 5, dadurch gekennzeichnet, daß man das bei der Umlösung verwendete Umlösewasser ganz oder teilweise dem Voransatz entnimmt.

## Claims

1. A process for the preparation of high-quality pure 3,3',4,4'-tetraaminobiphenyl (TAB) in high yield from crude TAB which has been prepared in a known manner by ammonolysis of 3,3'-dichlorobenzidine in the presence of copper powder and CuCl and has then been washed with ammonia water and water, which comprises redissolving the TAB, thus pretreated, in water in the presence of 0 to 5 % by weight of activated carbon and of 1 to 2 % by weight of a water-soluble reducing agent, relative in each case to the crude TAB, at temperatures of 100 to 140°C, under a protective gas atmosphere.

2. The process as claimed in claim 1, wherein the redissolution is carried out at temperatures of 105 to 125°C.

3. The process as claimed in at least one of claims 1 and 2, wherein the water-soluble reducing agent used is an alkali metal dithionite or alkali metal sulfite.

4. The process as claimed in at least one of claims 1 to 3, wherein the reducing agent used is sodium dithionite.

5. The process as claimed in at least one of claims 1 to 4, wherein the redissolution takes place under nitrogen.

6. The process as claimed in at least one of claims 1 to 5, wherein the redissolution water used in the redissolution is taken in whole or in part from the preceding batch.

## Revendications

1. Procédé pour la préparation de 3,3',4,4'-tétraaminobiphényle (TAB) de haute qualité, pur, avec un rendement élevé à partir du TAB brut, que l'on prépare par ammonolyse à partir de la 3,3'-dichlorobenzidine en présence de la poudre de cuivre et de CuCl et ensuite on lave à l'eau ammoniaquée et à l'eau, caractérisé en ce qu'on recristallise le TAB ainsi prétraité dans l'eau, en présence de 0 à 5% en poids de charbon actif et de 1 à 2% en poids d'un agent réducteur hydrosoluble, chaque fois par rapport au TAB brut, à des températures de 100 à 140°C, sous une atmosphère de gaz protecteur.

2. Procédé selon la revendication 1, caractérisé en ce qu'on met en oeuvre la recristallisation à des températures de 105 à 125°C.

3. Procédé selon au moins une des revendications 1 et 2, caractérisé ce qu'on utilise en tant qu'agent réducteur hydrosoluble un sulfite ou dithionite de métal alcalin.

4. Procédé selon au moins une des revendication 1 à 3, caractérisé en ce qu'on utilise en tant qu'agent réducteur de dithionite de sodium.

5. Procédé selon au moins une des revendications 1 à 4, caractérisé en ce qu'on effectue la recristallisation sous une atmosphère d'azote.

6. Procédé selon au moins une des revendications 1 à 5, caractérisé en ce que l'eau de recristallisation utilisée pour la recristallisation est reprise entièrement ou partiellement de la charge préliminaire.
